# EUROPEAN PATENT APPLICATION

(11) **EP 3 459 551 A1**
(43) Date of publication of application: **27.03.2019**
(21) Application number: 17306250.6
(22) Date of filing: 22.09.2017
(51) Int. Cl.: A61K 33/36, A61P 19/04, A61P 17/06, A61P 5/14, A61P 19/02, A61P 43/00, A61P 3/10

(54) **USE OF ARSENIC TRIOXIDE IN THE PREVENTION OR TREATMENT OF DISEASES ASSOCIATED WITH TYPE I INTERFERON SYSTEM ACTIVATION**

(71) Applicant: Medsenic, 67000 Strasbourg (FR)
(72) Inventor: The designation of the inventor has not yet been filed
(74) Representative: Santarelli

(57) **Abstract**

The present invention relates to the use of arsenic trioxide in the prevention and/or treatment of diseases associated with type I interferon system activation, in particular autoimmune diseases.

## Description

### FIELD OF THE INVENTION

The present invention relates to the use of arsenic trioxide in the prevention and/or treatment of diseases associated with type I interferon system activation such as in particular autoimmune diseases, and more particularly to the use of arsenic trioxide in the prevention and/or treatment of autoimmune diseases in patients with activated type I interferon system.

### BACKGROUND

Type I interferon (IFN) system was initially known for its antiviral effects but a large body of studies in humans increasingly implicates type I interferon in a list of autoimmune diseases including Systemic Lupus Erythematosus (SLE) and various autoimmune pathologies, suggesting a common underlying mechanism for their pathogenesis (Rönnblom et al., Clin. Exp. Rheumatol., 2016, 34 (Suppl.98):S1-S24); Domizio et al., Expert. Rev. Clin. Immunol., 2013, 9(3): doi:10.1586/eci.12.106; Crow et al., J. Immunol., 2014, 192(12):5459-5468).

Type I interferon is the largest family of interferons, which can be divided into five classes (IFN-α,-β, -ω, -ε and -κ). The IFN-α subgroup can be further divided into 12 subtypes, encoded by 13 highly homologous genes clustered on chromosome 9. The type I interferon system is defined as the type I IFNs themselves and all inducers, cells and molecules involved in the pathway leading to the production and effects of type I IFN. The most potent producer of type I IFN is the plasmacytoid dendritic cell (pDC) that can also activate CD4+ T cells, inducing either Th1, Th2 or Treg responses, depending on different signals of activation. Clinically, pDCs have been shown to be implicated in the pathogenesis of autoimmune diseases. These cells are activated by nucleic-acid containing complexes that selectively activate endosomal TLR7 or TLR9, which leads to prominent IFN production. Produced type I IFN proteins bind to and signal through the type I IFN receptor (IFNAR1 and IFNAR2), which activates a prominent Jak-Stat signaling pathway leading to expression of up to two thousand IFN stimulated genes (ISGs or IRGs for IFN-regulated genes), see the Interferome database (http://www.interferome.org/). The gene profile induced by type I IFN is today known as the IFN signature and the ISGs regulate a number of cellular functions besides inducing antiviral proteins.

Healthy individuals have almost no constitutive IFN-α production and do not have sustained IFN in their system. In contrast, abnormal IFN presence has been linked to Systemic Lupus Erythematosus (SLE) and various autoimmune pathologies.

The first autoimmune condition, where type I interferon is implicated is SLE, a systemic autoimmune disease with multiple organ involvement, characteristic antinuclear autoantibodies and formation of immune complexes (Tsokos et al., New Engl. J. med., 1979, 365(22), 2110-2121). There is a growing body of evidence linking type I interferons and SLE:
- Elevated type I interferons are found in the blood of SLE patients and the levels are associated with disease activity, flares and tissue injury especially of the skin, kidney and nervous system (Ytterberg et al., Arthritis Rheum., 1982, 25(4), 401-6; Hooks et al., N. Engl., J. Med., 1979, 301 (1):5-8).
   In childhood-onset SLE, the serum levels of IFN-α is positively correlated with circulating anti-dsDNA autoantibodies and SLE disease activity index scores (Postal et al., Clinics, 2012, 67(2):157-162).
- More than 50 % of adult patients with SLE display an IFN signature, not only in the blood but also in biopsied glomeruli from kidneys, which is connected to a more severe clinical picture with nephritis or haematological manifestations (Bennett et al., J. Exp. Med., 2003, 197:711-723; Baechler et al., Proc. Natl. Acad. Sci. USA, 2003, 100:2610-2615, Crow et al., Arthritis Res. Ther., 2003, 5, 279-287; Han et al., Genes Immun., 2003, 4:177-186; Yao et al., Hum. Genomics Proteomics, 2009, doi:10.4061/2009/374312).
   Paediatric lupus patients, who usually have a more severe disease compared to adult lupus patients, almost invariably display an IFN signature at early disease stages, suggesting that activation of the type I IFN system may be especially important in the initiation of the disease process (Bennett et al., J. Exp. Med., 2003, 197:711-723).
- Affected tissues in SLE patients tend to have an infiltration of pDCs, a key cellular player in the maintenance of chronic disease (Bave et al., J. Immunol., 2003, 171(6):3296-302).
- Autoantibodies specific for nucleic acids (anti-DNA and anti-RNA autoantibodies) that are common in lupus patients, are capable of inducing strong interferon secretion from pDCs (Bave et al., J. Immunol., 2003, 171(6):3296-302).
- Induction of DCs by IFN-α may drive the autoimmune response in SLE (Blanco et al., Science, 2001, 294, 15401543).
- Use of type I interferons as therapeutics in hepatitis C and oncology patients is associated with de novo appearance or exacerbation of existing autoimmune conditions including SLE (Ioannou et al., Arthritis Rheum., 2000, 43(7):1431-42).
- Polymorphism, or DNA sequence variants in important genes in the type I interferon pathway, including interferon regulatory factor 5 and tyrosine kinase 2, are associated with susceptibility to developing SLE (Sigurdsson et al., Am. J. Hum. Genet., 2005, 76(3):528-537).

It turns out that abnormal IFN presence is not limited to SLE but rather prevalent in various autoimmune pathologies:
- In psoriasis, a cutaneous autoimmune inflammatory condition, IFN signature is detected in the psoriatic plaques and induced IFN production further exacerbates the spread of lesions (Gilliet et al., Nat. Rev. Immunol., 2008, 8(8):594-606).
- Patients with Sjögrens syndrome, a disease of which the salivary and lacrimal glands are the targets of destructive autoimmune reactions, show an IFN signature, an important clue for understanding the disease pathogenesis (Gottenberg et al., Proc. Natl. Acad. Sci. USA, 2006, 103(8):2270-2275 Hjelmervik et al., Arthritis Rheum., 2005, 52:1534-1544; Baechler et al., Immunol. Rev., 2006, 210:120-137).
- Systemic sclerosis/scleroderma is a complex disease with features of extensive fibrosis and circulating autoantibodies against various cellular antigens. An activated type I IFN system with detectable IFN signature and IFN-α serum levels is associated with the vascular pathology and fibrotic process (Eloranta, Ann. Rheum. Dis., 2010, 69(7):1396-1402).
- Dermatomyositis, a severe multisystem autoimmune disease, manifests as muscle, skin and vasculature pathologies, which frequently associates with tissue calcification. Multiple studies revealed significant upregulation of type I IFN pathway in both adult and juvenile dermatomyositis patients (Baechler et al., Arthrit. Res. Ther., 2011, 13(6):249).
- Approximately 50 % of patients with rheumatoid arthritis (RA) display a peripheral blood IFN signature (van der Pouw Kraan et al., Ann. Rheum. Dis., 2007, 66: 1008-1014; Gardner et al., Virology, 2012, 425:103-112).

In addition, a genome wide survey identified a common gene set involved in the type I IFN pathway that are unanimously upregulated in patients with SLE, rheumatoid arthritis, myositis and systemic sclerosis (Higgs et al., Ann. Rheum. Dis., 2012, 70(11):2029-2036).

Collectively, these observations suggest that type I IFN pathway represents an important therapeutic target for autoimmune diseases in general. Diverse treatment strategies are being examined to downregulate IFN and dampen the progress of autoimmune diseases. High doses of glucocorticoids and hydroxychloroquine down-regulate the IFN signature and is widely used in SLE patients. More specific inhibitors of the type I IFN system exist: several monoclonal antibodies neutralizing IFN-α have been tested in clinical trials, resulting in partial decrease of the IFN signature and disease activity (Yao et al., Arthritis Rheum., 2009, 60, 1785-1796; Khamashta et al., Ann. Intern. Med., 2016). A more complete inhibition of the type I IFN family with a significant improvement in disease activity was achieved using anti-IFNAR, but at the cost of an increased frequency of viral infections (Furie et al., Arthritis Rheum., 2015, 67, S10). A broader therapeutic approach is to target the pDCs themselves with antibodies against BDCA-2 (Pellerin et al., EMBO Mol. Med., 2015, 7:464-476) or to use proteasome inhibitors which efficiently suppress production of IFN-α by TLR-activated pDCs (Ichikawa et al., Arthritis Rheum., 2012, 64:493-503). The optimal therapeutic target most probably depends on the mechanism(s) behind an increased type I IFN production or response in a specific subset of patients.

Given their pivotal role in type I IFN production and the pathogenesis of autoimmune diseases, the targeting of pDCs and in particular of type I IFN production by pDcCs represent a promising strategy for the treatment of autoimmune diseases, in particular in patients with activated type I IFN system.

Fowler's solution is a solution containing 1% potassium arsenite (KAsO₂), and was once prescribed as a remedy or a tonic. From 1845, Fowler's solution was used as a leukemia treatment. As some arsenical compounds are notably toxic and carcinogenic, with side effects such as cirrhosis of the liver, idiopathic portal hypertension, urinary bladder cancer, and skin cancers, Fowler's solution fell from use.
Interest in arsenic compounds returned with the use of Arsenic Trioxide (As₂O₃) as an anti-cancer ("antineoplastic" or "cytotoxic") chemotherapy. It has been approved by the US FDA for the treatment of acute promyelocytic leukemia that is unresponsive to "first line" agents, namely all-trans retinoic acid (ATRA). It has been shown that arsenic trioxide induces cancer cells to undergo apoptosis. The combination therapy of arsenic trioxide and all-trans retinoic acid (ATRA) has been approved by the U.S. Food and Drug Administration (FDA) for the treatment of acute promyelocytic leukemia.

In addition, ATO has been recently shown to have therapeutic potential in a number of autoimmune and inflammatory diseases, with a good safety profile (Bobé et al., Blood, 2006, 108, 13:3967 - 3975). Multiple mechanisms have been suggested to explain the efficacy of ATO, however little is known about its effect on human pDCs functions and phenotypes.

### SUMMARY

The inventors have shown that arsenic trioxide could be used in the treatment and the prevention of diseases associated with type I interferon system activation, such as in particular autoimmune diseases, and more particularly for treating and/or preventing autoimmune diseases in patients with activated type I interferon system. More specifically, they have shown that arsenic trioxide ATO affects the functional properties of pDCs. Non-lethal, therapeutic doses (low doses) of ATO reduced type I interferon, as well as other pro-inflammatory cytokines and chemokines release from activated pDCs. Moreover, ATO induced a suppressive phenotype of activated pDCs. Finally, ATO impaired pDCs ability to drive CD4+T cell proliferation and Th1/Th17/Th22 polarization. Overall, these findings suggest that low dose ATO is a promising agent for the treatment and the prevention of diseases associated with type I interferon system activation such as autoimmune diseases, and more particularly for the treatment and the prevention of autoimmune diseases in patients with activated type I interferon system.

### DETAILED DESCRIPTION

The present invention relates to arsenic trioxide for use in the prevention or treatment of diseases associated with type I interferon system activation.

As used herein, the term "treat" or "treating" and their derivatives includes substantially inhibiting, slowing or reversing the progression of a condition, substantially ameliorating symptoms of a condition or substantially preventing the appearance of symptoms of a condition.

The term "prevent" and all variations of this term is intended to mean the countering in advance of symptoms of a condition. In this case it is understood that the composition is applied prior to the observation of the first symptoms.

The terms "ameliorate" and "amelioration" relate to the improvement in the treated subject's condition brought about by the compositions and methods according to the invention.

As used herein, the expression "disease associated with type I interferon system activation" is intended to mean a disease, in particular an autoimmune disease, whose pathophysiology is driven or enhanced by type I interferon system activation.

In some embodiments, arsenic trioxide is for use in the prevention or treatment of diseases, associated with type I interferon system activation, in particular autoimmune diseases, in patients with activated type I interferon system.

As used herein, the term "patient" refers to a subject affected with a disease or condition to be treated by ATO administration. The subject may be human or non-human. In some preferred embodiments, said patient is a human subject.

As used herein, the expression "patients with activated type I interferon system" is intended to mean patients, in particular autoimmune disease patients, whose pathophysiology of disease is partially or totally driven by type I interferon system activation.

The type I interferon system is defined herein as the type I IFNs themselves and all inducers, cells and molecules involved in the pathway leading to the production and effects of type I IFNs.

Activation of the type I interferon system includes in particular:
(i) increased level of type I interferon protein(s), preferably IFN-α protein, in the peripheral blood, plasma or serum, and/or
(ii) increased expression level of one or more type I interferon stimulated genes (ISGs) in the peripheral blood and/or affected tissue(s) or organ(s) of the patient to whom arsenic trioxide is intended to be administered.

Interferon protein level is measured using standard measurement methods and kits that are well-known in the art. For example, type I IFN protein level such as IFN- α protein level may be measured by ELISA using specific monoclonal antibodies.

Type I interferon stimulated genes are well-known in the art and disclosed in public data base such as the Interferome database (http://www.interferome.org/). In some preferred embodiments, the ISGs which are activated (IFN signature) comprise one or more of HERC5, EPSTI and CMPK2 genes. These three genes are included in the IFN signature in SLE patients.

The expression level of type I interferon stimulated gene(s) is determined either by measuring the level of transcripts (mRNA) from said genes or by measuring the level of proteins produced from the transcripts of said genes. Preferably, the expression level of IRGs is determined by measuring the level of transcripts from IRGs.

Type I interferon regulated or stimulated gene expression is measured using standard measurement methods and kits that are well-known in the art. For example, qPCR assays with primers and fluorescent dye-labelled probes for IRGs are available in the art.

The tissue(s) or organ(s) which are analyzed can be from a biopsy. A biopsy can be from any organ or tissue. The biopsy is for example a biopsy of muscle, skin, kidney, brain, heart, eye, joint, lung, gland, digestive tract or blood vessel.

The level of type I IFN protein or IRG expression is usually compared to a reference level from the same tissue or organ in a healthy individual used as control. However, the sole detection of type I Interferon protein or transcripts or proteins from IRGs in the blood, tissue or organ may be indicative of type I interferon system activation. Indeed, as mentioned above, healthy individuals have almost no constitutive IFN-α production and do not have sustained IFN in their system.

According to the invention, arsenic trioxide is used for inhibiting or repressing the type I interferon system in the treated patient. In particular, arsenic trioxide is used for reducing the level of type I interferon protein, preferably IFN-α protein, in the peripheral blood, plasma or serum, and/or for reducing the expression level of one or more type I interferon stimulated genes (ISGs) in the peripheral blood and/or affected tissue(s) or organ(s) of the treated patient.

In some embodiment, the diseases associated with type I interferon system activation are autoimmune diseases such as for example: alopecia areata, autoimmune hemolytic anemia, autoimmune hepatitis, dermatomyositis, diabetes (type 1), some forms of juvenile idiopathic arthritis, glomerulonephritis, Graves' disease, Guillain-Barré syndrome, idiopathic thrombocytopenic purpura, myasthenia gravis, some forms of myocarditis, multiple sclerosis, pemphigus/pemphigoid, ernicious anemia, polyarteritis nodosa, polymyositis, primary biliary cirrhosis, psoriasis, rheumatoid arthritis, scleroderma/systemic sclerosis and vascularites, Sjögren's syndrome, systemic lupus erythematosus, some forms of thyroiditis such as Hashimoto's thyroiditis, some forms of uveitis, vitiligo and granulomatosis with polyangiitis (Wegener's), coeliac disease, Crohn's disease, ulcerative colitis, Behcet's disease and Graft-Versus-Host-Disease (GVHD).

In some preferred embodiments, said autoimmune diseases are selected from the group consisting of: systemic lupus erythematosus (SLE) including childhood-onset and adult-onset lupus erythematosus and its nephretic forms, scleroderma/systemic sclerosis and vascularites, autoimmune myopathies such as dermatomyositis and polymyositis, psoriasis, rheumatoid arthritis, Sjögren's syndrome, type 1 diabetes, multiple sclerosis, Behcet's disease, Hashimoto's thyroiditis and GVHD; preferably from the group consisting of: systemic lupus erythematosus (SLE) including childhood-onset and adult-onset lupus erythematosus and its nephretic forms, scleroderma/systemic sclerosis, autoimmune myopathies such as dermatomyositis and polymyositis, psoriasis, rheumatoid arthritis, Sjögren'ssyndrome] and GVHD; more preferably from the group consisting of: systemic lupus erythematosus (SLE) including childhood-onset and adult-onset lupus erythematosus and its nephretic forms, scleroderma/systemic sclerosis, dermatomyositis, psoriasis, rheumatoid arthritis and Sjögren'ssyndrome.

In some preferred embodiments, said diseases are at an early stage, preferably early-stage autoimmune diseases, such as for example early-stage (childhood-onset or adult-onset) lupus erythematosus, early-stage scleroderma/systemic sclerosis, early-stage dermatomyositis, early-stage psoriasis, early-stage rheumatoid arthritis or early-stage Sjögren'ssyndrome.

As used herein, "early-stage disease ", in particular early-stage auto-immune disease is intended to mean the time of appearance of clinical symptoms.

In some preferred embodiments, said diseases, preferably autoimmune diseases do not comprise a lymphoproliferation (i.e. aberrant or excessive lymphoproliferation) such as a for example a lymphoproliferative syndrome.

As used herein, "lymphoproliferation" is intended to mean aberrant clonal cell proliferation of cells from lymphoid tissue.

In some embodiments, arsenic trioxide is used in the prevention or treatment of autoimmune diseases as defined above, in patients with activated type I interferon system, as defined above.

In the various embodiments of the present invention, arsenic trioxide is administered in the form of a pharmaceutical composition comprising a therapeutically effective amount of arsenic trioxide. This effective amount intended to be administered daily is a dose of 0.01 to 5 mg/kg of body weight, preferably 0.05 to 0.5 mg/kg, even more preferably 0.10 to 0.40 mg/kg.

The various embodiments of the invention encompass the use of arsenic trioxide or any pharmaceutically acceptable salt thereof.

This composition may also comprise a pharmaceutically acceptable carrier or excipient. Depending on the applications, it will be in a form that is suitable for any appropriate route of administration, e.g., oral, dermal, parenteral, intraperitoneal administration.

The carrier may be of very varied type, according to the form of the preparation used for the administration, in particular oral, dermal or parenteral (lozenge, capsule, powder, intravenous liquid or encapsulated injection, infusion or ingestion).

For oral administration, the pharmaceutical preparation may be preferably but not only in liquid form, for example in solution, in the form of a syrup or of a suspension, or in the form of a powder intended to be re-dissolved. Such a liquid preparation can be prepared according to suitable techniques with pharmaceutically acceptable excipients such as suspending agents (for example: sorbitol, cellulose derivatives), emulsifiers (for example: lecithin or acacia), non-aqueous transporters (for example: fractionated vegetable oil), and preserving agents (for example: sorbic acid).

When solid oral preparations (lozenge, powder, capsule, tablet) are involved, the pharmaceutical compositions are prepared using suitable excipients such as binding agents (corn starch, polyvinyl pyrrolidone, hydroxypropylmethylcellulose), filling agents (lactose, microcrystalline cellulose), lubricants (magnesium stearate, talc, silica) or swelling agents (sodium lauryl sulfate).

In one embodiment, the arsenic trioxide is not in the form of Fowler's solution.

For administration by inhalation, the compounds are typically included in a preparation of the aerosol type using a suitable gas.

The arsenic trioxide can also be formulated for parenteral administration, for example by continuous or non-continuous injection. The liquid media are generally similar (water, glycol, oil, buffer) to those used for oral preparations.

The formulations by injection can be provided in the form of dosage units (ampoules, vials, mini-containers) with a suitable protective agent. These compositions to be injected can also be in the form of a suspension, a solution or an emulsion and can contain stabilizers and/or dispersing agents. The active principle can also be prepared in the form of a powder or a cream with a suitable transporter or encapsulated or incorporated in a suitable transdermal preparation or patch. The invention also provides packs or kits comprising at least one container containing the arsenic trioxide in a pharmaceutically acceptable form. For example, the arsenic trioxide may be in the form of a pharmaceutically acceptable solution, such as a saline solution, an ose-containing solution or a sterile buffer solution; the kit may also comprise suitable means for injection, such as a sterile packaged syringe.

The preferred ways of administration are oral-with adequate specific release system in the intestinal compartment, intravenous- soluble arsenic trioxide-, as traditionally performed for cellular targets present in the blood (e .g. dendritic cells) or transdermal.

The therapeutic dose and number of applications, injections or oral and other ways of administration used in the treatment of diseases associated with type I interferon system activation is variable according to the type of chronicity, symptoms seriousness and the conditions (routes of administration) of treatment. The dose, and where appropriate the frequency, are to be adjusted as a function in particular of age and of bodyweight, in addition to severity of disease and its stage. Given the potential toxicity of the arsenic, the dosage and the duration of the treatment are determined in an appropriate manner, according to the seriousness of the disease and the long-lasting nature, or otherwise, of the recovery, it being possible for the treatment to last from a few days to a few months/ years until complete or at least partial recovery is achieved. The formulation is typically administered daily, for a period of 10 to 60 days. Several successive treatments may be carried out, of the order of one month apart.

Administration of arsenic salts is recommended to aim at reaching a quick high value in the circulatory system at the start of treatment, so as to inhibit the type I interferon production and to be delivered at a dose of 0.25-0.35 mg /kg/day over a few more days for sustained inhibition and saturation of arsenic sensitive pathways to interferon production, eventually followed - if found necessary-by a maintenance treatment once every month over a two years follow up.

In addition, the arsenic trioxide may be administered, where appropriate, with other active principles that participate in the treatment of the targeted diseases. Thus, mention may be made of the use of the arsenic in combination with corticosteroids such as prednisone or drugs such as methotrexate, for treating autoimmune syndromes and/or chronic inflammatory pathologies or any other active component which may occur to add or synergize to the prophylactic, therapeutic or curative action of arsenic salts. Other synergic drugs may be added to a treatment regimen, such as immunoregulative drugs or drugs intensifying or giving a better targeting for arsenic trioxide.

According to another aspect, the invention relates to a method for treating, in particular in humans, a disease associated with type I interferon system activation, as defined above, comprising the administration to the patient of a therapeutically effective amount of arsenic trioxide. The invention relates to a method of treatment comprising the administration of 0.01 to 5 mg, preferably 0.05 to 0.5 mg, even more preferably 0.10 to 0.40 mg of arsenic trioxide per kilogram of body weight per day. The administration may be oral, transdermal, parenteral, intraperitoneal, intravenous or via any other appropriate route. According to a preferred embodiment of the method, arsenic trioxide is administered to a patient with activated type I interferon system, preferably an autoimmune disease patient with activated type I interferon system, as defined above.

According to another aspect, the invention relates to a method for inhibiting or repressing the type I interferon system in a patient suffering from an autoimmune disease, in particular a human, comprising the administration to the patient of an effective amount of arsenic trioxide.

In one embodiment of the use or method of the invention, the patient does not suffer from cancer at the time of arsenic trioxide treatment, in particular from acute promyelocytic leukemia.

In another embodiment of the use or method of the invention, the patient does not suffer from lymphoproliferation; preferably the patient does not suffer from lymphoproliferation and from cancer, in particular acute promyelocytic leukemia.

Other subjects and advantages of the invention will emerge from the following examples.

### EXAMPLES

### Materials and Methods

pDCs were isolated from fresh peripheral blood mononuclear cells (PBMCs) by magnetic selection. Cell viability, phenotype and intracellular cytokine analyses were performed with flow cytometry. ELISA was used to analyze the cytokine concentration in supernatants.

### Results

First, the effect of ATO on the viability of human pDCs was investigated. After 24h of culture, Fixable Viability Dye test showed that ATO decreased the viability of pDCs in a dose-dependent manner. Equal or over 1µM of ATO significantly decreased viability of both non-activated and activated pDCs. (% of living pDCs: 56.36±6.44% with 0µM vs 32.02±7.12% with 1µM of ATO, p=0.039)

Then, the effect of low dose ATO (corresponding to a non-lethal, therapeutic dose) on cytokine and chemokine secretion by pDCs after TLR9 activation was studied. ELISA test of supernatants of sorted pDCs cultured with ATO for 24h were studied (n=4). Results showed that ATO significantly reduced IFN-α secretion (53.24±6.61ng/ml with 0µM vs 25.81±7.70ng/ml with 0.5µM of ATO, p=0.004), as well as other pro-inflammatory cytokines and chemokines TNF-α (6.23±0.92ng/ml with 0µM vs 3.99±0.79ng/ml with 0.5µM of ATO, p=0.005), IL-6 (2.31 ±0.39ng/ml with 0µM vs 1.66±0.32ng/ml with 0.5µM of ATO, p=0.029), and CXCL10 (13.06±0.98ng/ml with 0µM vs 9.44±0.97ng/ml with 0.5µM of ATO, p=0.036).

Given the above results, it was addressed whether activated pDCs phenotype could be similarly impacted by ATO treatment. Results showed a significant decrease of relative fluorescence intensity (RFI) of CD80, CD86, and HLA-DR expressions, and % of CD86+ and CCR7+ pDCs after 24h culture with non-lethal doses of ATO. Moreover, it was found an increase of RFI for PD-L1 expression (p=0.09) and a significant increase of PD-L1+ pDCs (61.44±2.37% with 0µM vs 73.27 ±3.72% with 0.5µM of ATO, p=0.005, n=4), demonstrating that ATO can negatively impact the maturation of human pDCs.

In order to evaluate whether ATO can influence the capability of pDCs to activate T cells, mixed lymphocyte reaction (MLR) of pDCs and CD4+ naïve T cells were performed. It was observed that when CD4+ naïve T cells were co-cultured with ATO-treated activated pDCs, their proliferation was significantly decreased, compared to MLRs with non-treated activated pDCs (% of proliferating T cells: 44.97±2.53% with 0µM vs 37.98±2.11% with 0.5µM of ATO, n=4).

In addition, the effect of ATO treatment on pDC capacity to polarize naïve CD4+ T cells was assessed. Intracellular staining showed a significant decrease of IFN-γ (41.13% vs 29.09%), TNF-α (47.10% vs 35.40%), and IL-22 (11.67% vs 7.22%) positive cells in T cells when the pDCs have been previously treated with ATO. Moreover, other cytokines were measured by ELISA and the results revealed a significant decrease of IL-17, IL-22 concentrations in the supernatants of MLR, while there was no change in IL-10 concentration.

In conclusion, these results demonstrate that ATO affects the functional properties of pDCs. Non-lethal doses of ATO reduced type I interferon, as well as other pro-inflammatory cytokines and chemokines release from activated pDCs. Moreover, ATO induced a suppressive phenotype of activated pDCs. Finally, ATO impaired pDCs ability to drive CD4+T cell proliferation and Th1/Th17/Th22 polarization. Overall, these findings suggest that low dose ATO, corresponding to a non-lethal, therapeutic dose, is a promising agent for the treatment of autoimmune disorders and graft-versus-host disease.

## Claims

1. Arsenic trioxide for use in the prevention or treatment of diseases associated with type I interferon system activation.

2. Arsenic trioxide for the use according to claim 1, wherein said diseases are auto-immune diseases.

3. Arsenic trioxide for the use according to claim 2, which is for use in patients with activated type I interferon system.

4. Arsenic trioxide for the use according to claim 3, wherein said patients have elevated level of Interferon-α protein in the peripheral blood and/or elevated expression level of one or more type I interferon stimulated genes in an affected tissue or organ.

5. Arsenic trioxide for the use according to any one of claims 2 to 4, wherein said autoimmune diseases are selected from the group consisting of: systemic lupus erythematosus (SLE), systemic sclerosis and vascularites, dermatomyositis, polymyositis, psoriasis, rheumatoid arthritis, Sjögren'ssyndrome type 1 diabetes, multiple sclerosis, Behcet's disease, Hashimoto's thyroiditis, and GVHD.

6. Arsenic trioxide for the use according to any one of claims 1 to 5, wherein said diseases are at an early-stage of these diseases.

7. Arsenic trioxide for the use according to any one of claims 1 to 6, wherein said diseases do not comprise a lymphoproliferation.

8. Arsenic trioxide for the use according to any one of claims 1 to 7, which is for use by oral, transdermal or intravenous administrations.
